# EUROPEAN PATENT SPECIFICATION

(11) **EP 1 558 304 B1**
(45) Date of publication and mention of the grant of the patent: **05.08.2009**
(21) Application number: 03777770.3
(22) Date of filing: 22.10.2003
(51) Int. Cl.: A61M 5/142

(54) **DRUG INFUSION SYSTEM WITH MULTIPLE MEDICATIONS**
INFUSIONSSYSTEM FÜR MEHRERE MEDIKAMENTE
SYSTEME DE PERFUSION PERMETTANT L'ADMINISTRATION DE PLUSIEURS MEDICAMENTS

(30) Priority: 22.10.2002 US 278767
(43) Date of publication of application: 03.08.2005
(73) Proprietor: MEDTRONIC, INC., Minneapolis MN 55432-5604 (US)
(72) Inventor: JASPERSON, Keith, E., Andover, MN 55304 (US); VALINE, Thomas, J., Spring Lake Park, MN 55432 (US); WAHLQUIST, Frederic, J., R., Champlin, MN 55316 (US)
(74) Representative: Hughes, Andrea Michelle
(86) International application number: PCT/US2003/033416
(87) International publication number: WO 2004/037324

(56) References cited:
- WO-A-02/072178
- WO-A-03/004034
- US-A- 5 317 506
- US-A1- 2002 019 606

## Description

This invention relates to drug infusion systems and, in particular, drug infusion systems that are programmable by a medical professional.

Drug infusion systems dispense fluid medication, containing a drug, to a patient. Some drug infusion systems are portable, allowing a patient to receive fluid medication while remaining mobile. In addition, some drug infusion systems are implantable to more effectively and less obtrusively dispense such fluid medication to a patient.

Implantable devices and techniques for treating a patient by drug infusion are well known in the prior art. For instance, U.S. Patent No. 5,782,798, Rise, entitled Techniques For Treating Eating Disorders By Brain Stimulation and Drug Infusion; and U.S. Patent No. 5,814,014, Elsberry et al, Techniques of Treating Neurodegenerative Disorders by Brain Infusion, each assigned to Medtronic, Inc., Minneapolis, Minnesota, disclose such devices and techniques.

Another example of a drug infusion device is shown in U.S. Patent No. 3,527,220, Summers, entitled Implantable Drug Administrator, an implantable drug administrator having a refillable bladder which can be filled with a drug and a pump for selectively pumping the drug from the bladder into any desired area of the body. The administrator includes an indicator for indicating when the desired amount of the drug has been injected.

In U.S. Patent No. 3,951;147, Tucker et al, entitled Implantable Infusate Pump, a rechargeable infusate pump for implantation in the human body can be refilled periodically by injection through an inlet septum under the skin. A conduit conducts fluid to an infusion site in the body. The pump outlet includes a special controller flow controller which is able to very accurately meter the infusate to the selected body site.

A problem with these implantable drug infusion devices is that there is no way to provide a simple external means to select the dosage amounts and intervals from a wide range of possible doses and intervals, and verify that a desired change had been made.

U.S. Patent No. 4,146,029, Ellinwood, Self-Powered Implanted Programmable Medication System and Method, discloses a device and method for dispensing medication internally of the body utilizing an implanted system which includes medication storage and dispensing control circuitry having control components which may be modified by means external of the body being treated to control the manner of dispensing the medication within such body. In particular, extracorporeal control means may provide some measure to achieve selected medication programs corresponding to particular codes.

U.S. Patent No. 4,692,147, Duggan, Drug Administration Device, assigned to Medtronic, Inc., Minneapolis, Minnesota, discloses an implantable drug administration device which can be non-invasively programmed to change both the dosage amount and the dosage interval. Verification of the received dosage and interval commands is achieved by means of an audio transducer which is attached to the device case.

The implantable drug administration device described in Duggan allows a medical professional to program to the delivery rate of a drug contained in the reservoir of the device over a specified interval.
US Patent Application publication No. 2002/019606 discloses a drug infusion system having an implantable drug delivery module with a set of operating parameters stored in its memory.

Not infrequently, a medical professional prescribes more than one drug to be used in an implantable drug infusion device. More than one active ingredient present in the reservoir of the implantable infusion device increases programming difficulties substantially. Not only must the medical professional program the drug infusion device to perform a series of programmed steps in order to deliver one drug to the patient, the medical professional must take into account the effect of creating or modifying a program for one of the drugs on the delivery of all other drugs also contained within the same reservoir of the drug infusion device. If the medical professional changes the delivery rate of the drug infusion device to increase the dose of one drug to be delivered to the patient in a period, that change will also increase the dose of all other drugs that are also contained in the same reservoir. With a complex dosing regimen and a plurality of active drugs, the danger for confusion and error is significant.

The present invention is defined in claim 1. It provides a drug infusion system capable of delivering a fluid medication to a patient at a flow rate under direction of a medical professional, said fluid medication consisting of a plurality of drugs to be administered from a reservoir, each of said plurality of drugs having a concentration, said drug infusion system comprising:
a drug delivery module capable of delivering said fluid medication to said patient from said reservoir; and
a controller allowing said medical professional to specify a dose per unit time of one of said plurality of drugs; wherein
means are provided for determining a flow rate of another of said plurality of drugs as a function of said dose per unit time of said one of said plurality of drugs and said concentration of said another of said plurality of drugs; and
said controller communicating said flow rate of said another of said plurality of drugs to said medical professional.

In a preferred embodiment, the concentration of each of the plurality or drugs is stored in the drug delivery module.

In a referred embodiment, the controller communicates the dose for the secondary drug per unit time to the medical professional via a display.

In a preferred embodiment, the drug delivery module is implantable.

In a preferred embodiment, the controller communicates the dose for the secondary drug per unit time to the medical professional via a display.
Preferred embodiments will now be described, by way of example only, with reference to the drawings.

Figure 1 is a schematic view of a patient with a drug infusion device implanted within the patient's body.

Figure 2 is a block diagram of a drug infusion device of the present invention;

Figure 3 is a block diagram illustrating the random access memory and register layout of a portion of drug infusion device of the present invention;

Figure 4 is an illustration of a drug entry display provided to a programmer of the drug infusion system of the present invention;

Figure 5 is another illustration of a drug entry display provided to a programmer of the drug infusion system of the present invention;

Figure 6 is an illustration of a drug delivery display provided to a programmer of the drug infusion system of the present invention showing simple continuous mode programming;

Figure 7 is another illustration of a drug delivery display provided to a programmer of the drug infusion system of the present invention showing simple continuous mode programming; and

Figure 8 is an illustration of a drug delivery display provided to a programmer of the drug infusion system of the present invention showing flex mode programming.

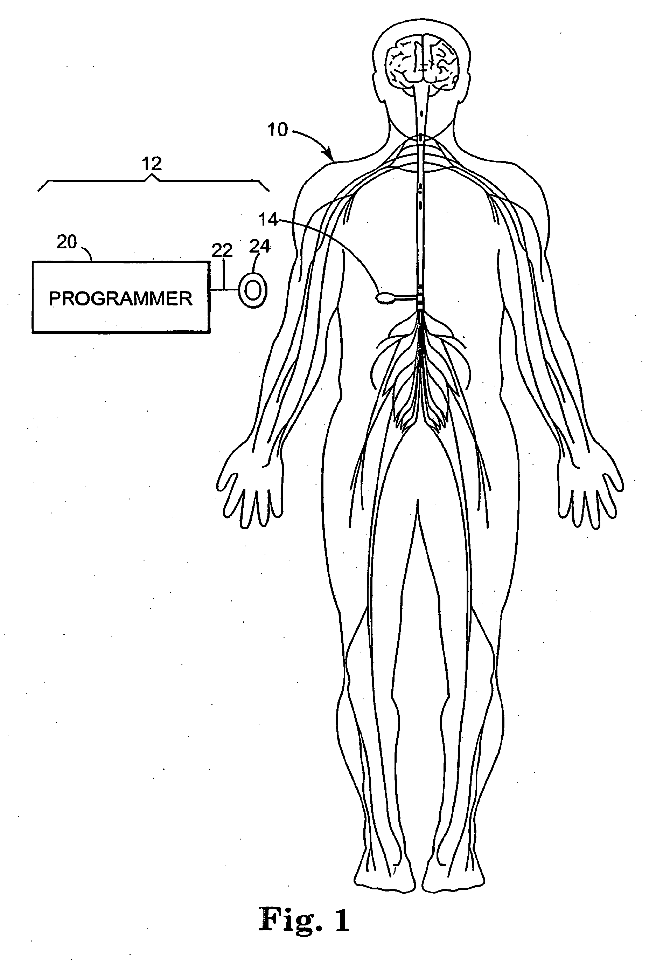

Figure 1 is a schematic view of a drug infusion system 12 of the present invention. Implantable drug infusion device 14 is shown implanted within the body of patient 10. Drug infusion device 14 is programmable through a telemetry link from programmer 20, which is coupled via a conductor 22 to a radio frequency antenna 24. Methods of communicating, using radio frequency telemetry, with implanted treatment devices in order to program such implanted drug infusion devices, are well known in the art.

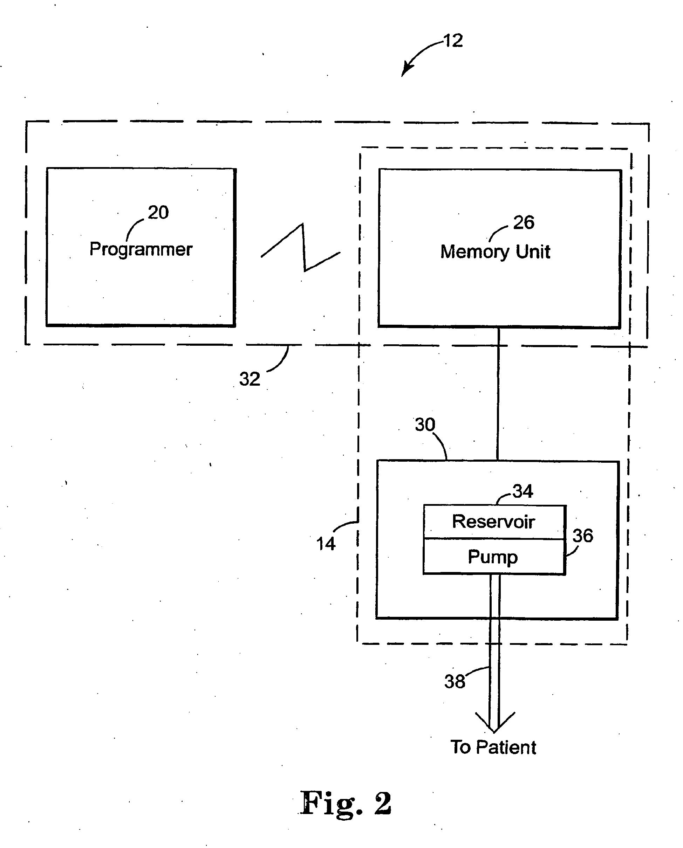

Figure 2 is a block diagram of the drug infusion system 12 having an implantable drug infusion device 14. Drug infusion device 14 consists of an internal memory unit 26 containing memory and registers and circuitry which provides internal drug delivery instructions to drug delivery module 30. External programmer 20 acts as an input-output device for drug infusion system and also provides computational support for memory unit 26. Memory unit 26 and programmer 20, operating together, function as a controller 32 controlling drug delivery module 30 in the delivery of fluid medication to patient 10. Drug delivery module 30 has a reservoir 34 for holding the fluid medication to be infused and pump 36 coupled to patient 10 through catheter tubing 38. Such drug delivery modules 30 are well known in the art.

Memory unit 26 receives programming information, via telemetry, from programmer 20 through conventional means. Programming information, once stored in memory unit 26, provides the dosing regimen to be performed by drug delivery module 30.

Memory unit 26 stores information concerning aspects of the operation of drug infusion device 14. Memory unit 26 may, for example, store information about patient 10 including name, address and contact information. In addition, memory unit 26 may store information about the drug delivery module 30 including pump 36 and catheter 38. Among the settings that may be stored are the model number and serial number of pump 36, the volume of reservoir 34, battery condition and information about catheter 38; including the length of all sections of catheter 38. Information about the calibration of pump 36 may also be stored. During the pumping operation, controller 32 (preferably through programmer 20) also calculates, or otherwise determines, the volume of fluid medication remaining in reservoir 34.

In order to perform its function, memory unit 26 also stores information about each drug contained in reservoir 34, including the drug name, or other identifier, and the concentration of the drug in the overall volume of fluid medication contained in reservoir 34. Typically, this data is entered at the time that drug infusion device 14 is loaded with fluid medication.

Throughout this description, while it is contemplated that any or all of the calculations performed by controller 32 could be performed in either programmer 20 or memory unit 26, it is recognized that drug delivery device 14, being an implantable device, will have a limited amount of processing power and energy source. Therefore, it is preferred that the calculation referred to as being performed by controller 32 (encompassing both programmer 20 and memory unit 26) actually be performed by programmer 20, in order to control the precious resources of implantable drug infusion device 14. At the same time, it is preferred that information concerning implantable drug infusion device 14, patient 10, all drug contained in reservoir 34 and the drug regimens implemented, all be stored in memory unit 26 so as to be available no matter which of a plurality of programmers 20 may be operationally coupled with implantable drug infusion device 14 to form drug infusion system 12.

Alternatively, the amount of drug, e.g., in micrograms, can be entered into controller 34 when drug infusion device is loaded with fluid medication. That information, along with the also known volume of fluid medication, e.g., 12 milliliters, allows controller 34 to calculate the concentration of the drug. In the example given above, if the volume of fluid medication is 12 milliliters and the amount of drug is 20 micrograms, then the concentration the drug 1.666 micrograms per milliliter (20 micrograms divided by 12 milliliters).

Drug infusion device 14 may also contain (and be programmed for) more than one drug. Typically, a multiple drug prescription, the drug cocktail containing multiple drugs, or an active drug and a neutral agent such as saline, is premixed and then injected into the implanted drug infusion device. The concentration of each of the drugs contained in the drug cocktail is known, or the amount of each of the drugs contained in the drug cocktail is known, or a combination of the above. The requisite information for each of the drugs contained in the drug cocktail are entered into memory unit 26 in the same way as described above with respect to one drug.

In a preferred embodiment, the patient information, pump information and the drug information is all stored in memory unit 26 located in an implanted drug infusion device 14. While all of this information readily available from within implanted drug infusion device 14, a medical professional may use any applicable programmer 20, at any time and any location, to read information from implanted drug infusion device 14 and to program drug infusion device 14.

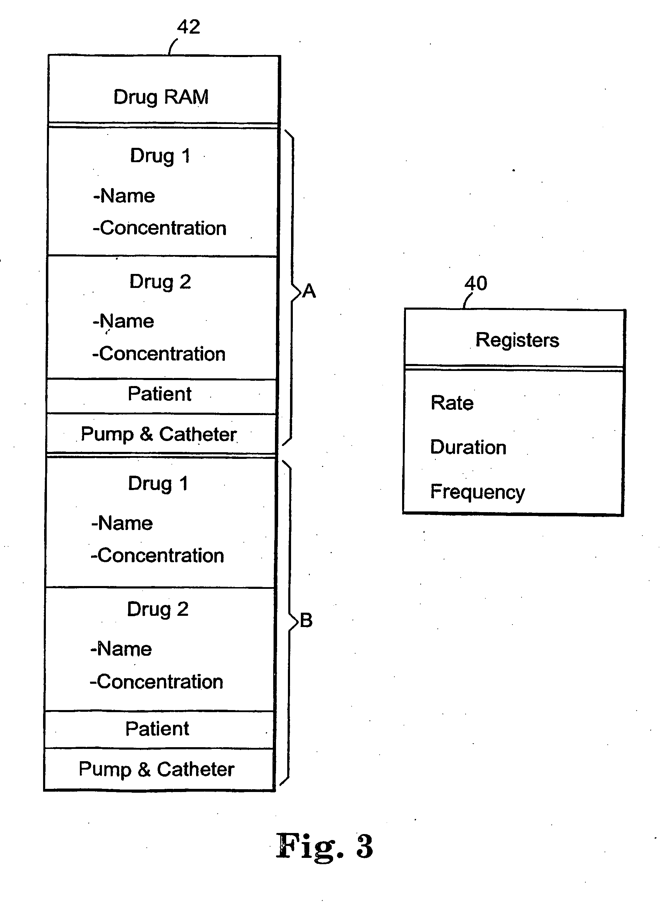

Figure 3 illustrates a preferred manner of storing such information within memory unit 26 of drug infusion device 14. Registers 40 contain information concerning programmed drug regimens, including the number of steps, their frequency (if repetitive), duration and pumping rate. Such information is generic to all drugs contained in reservoir 34 irrespective of the nature of the drug or drugs contained in reservoir 34 or the number of drugs contained in reservoir 34. In general, the information contained in registers 40 represents the information which applies whichever drug is contained in reservoir 34 or applies equally to all drugs in reservoir 34. For example, the delivery rate represents the programmed rate at which pump 36 delivers fluid medication to patient 10. Since all of the drugs present in reservoir 34 of drug infusion device 14 are delivered to patient from common reservoir 34 at whatever rate pump 36 is programmed, all drugs are delivered to patient 10 at precisely the same rate. Hence; information about delivery rate, pumping rate, may be stored in registers 40 and can be common to all drugs contained in drug infusion device 14.

Drug RAM (random access memory) 42 holds information about each of the drugs which are contained in reservoir 34 of drug infusion device 14. As noted above, such drug information includes the name, or other identifier, of each individual drug as well as the concentration of the drug in total fluid volume of fluid medication and/or the amount of such drug contained in reservoir 34.

In a preferred embodiment, drug RAM 42 is separated into two at least two parts, labeled part A and part B. Parts A and B of drug RAM 42 are identical and each contains exactly the same type of information, although, of course, the data contained in each individual memory location or locations may differ. Thus, parts A and B of drug RAM 42 are completely redundant. However, only either part A or part B of drug RAM 42 is active at any one time.

The information in part A when active, for example, of drug RAM 42, in conjunction with the information contained in registers 40, specifies the operating parameters and controls the operation of drug delivery module 30 and, therefore, delivers the proper amount of fluid medication to patient 10 at the proper time. In this case, the operating parameters in part B of drug RAM 42 are inactive and do not control the operation of drug infusion device 14. Conversely, when the operating parameters stored in part B of drug RAM 42 are active, the information contained therein, along with the information contained in registers 40, controls the operation of drug infusion device 14 and the operating parameters stored in part A of drug RAM 42 are inactive and do not control any aspect of the operation of drug infusion device 14. Thus, parts A and B are completely redundant and alternatively control the operation of drug infusion device 14.

When new information about the drugs contained in reservoir 34 of drug infusion device 14, patient information or information about pump 36 and/or catheter 38 is written to drug RAM 42, it is written to the part of drug RAM 42 which is not, at that time, active.

Mainly due to the amount of information which may need to be written to the inactive portion of drug RAM 42, e.g., information about multiple drugs including their name and concentration, the writing of such information may need to be performed in separate write steps or, in other words, in separate packets of information. Because the information is not written in a simple step, or in a single packet, there exists the possibility that the writing process may be interrupted. This could occur, for example, if communication with implantable drug infusion device 14 was lost due to movement of patient 10 or of programmer 20 or could occur if battery power were lost to programmer 20.

Since the information about drugs, patient and pump 36 and/or catheter 38 are written to the portion of drug RAM 42 which is not currently active, the inactive portion of drug RAM 42 serves as a shadow RAM to hold such information until the entire writing process can be finished. Registers 40 can then be updated to transfer control of drug infusion device 12 from the previously active portion of drug RAM 42 to the newly written and previously inactive portion of drug RAM 42.

Information about the rate, duration and frequency of each step of drug delivery programmed into drug infusion device 12 is contained in registers 40. The rate, duration and frequency information contained in registers 40, along with the information contained in drug RAM 42 and information contained in registers 40 on which portion of drug RAM 42 is active, determine the operation of drug infusion device 12. In a preferred embodiment, new information written into registers 40 concerning rate, duration, frequency and which portion of drug RAM 42 is active, is written as a single step. In other words, this information is written as a single packet of data. Thus, there is no possibility that some of the information will be written and the writing process will be interrupted. Thus, new information, if needed, is written into the inactive portion of drug RAM 42 first and then new information, if needed, is written into registers 40 and control is transferred from the previously active portion of drug RAM 42 to the previously inactive portion of drug RAM 42. This stepped process enables information from a plurality of writing steps to be transferred to the memory which controls drug infusion device 12 without the danger that an interruption in writing process will result in only a portion of the intended new information controlling drug infusion device 12.

It is also to be recognized and understood that while registers 40 and drug RAM 42 of memory unit 26 have been described, in a preferred embodiment, as having two parts, namely parts A and B, that the same principles apply and registers 40 and drug RAM 42, or either of them, may be separated into more than two parts with equally advantageous operating results.

The operating parameters stored in each part (part A and part B) of drug RAM 42 of memory unit 26 may be again divided into separate areas. Each part of drug RAM 42 contains information relating to each of the drugs contained in reservoir 34. For example, if two drugs are contained in reservoir 34 (and part A is active), then part A of drug RAM 42 will be divided into sections. There is a section devoted to information about drug one and a section devoted to information about drug two. And, of course, drug RAM 42 may be separated into any multiple of parts, at least one for each of the number of drugs which are contained, or which may be contained, in reservoir 34.

If more than one drug is prescribed for drug infusion system 12, the proper amount of each drug will typically be pre-mixed before insertion into reservoir 34 of implantable drug infusion device 14. Each drug in the mixture will have a concentration, i.e., an amount of each drug compared to the overall volume of fluid medication contained, to be contained, in reservoir 34. At or near the time that the drug mixture containing the multiple drugs is inserted into reservoir 34, usually through a syringe for an implantable drug infusion device 14, data concerning all of the drugs contained in fluid medication is entered into memory unit 26.

Generally, one of the drugs contained in fluid medication is the primary drug. The primary drug is main drug around which the prescription drug mix, or drug cocktail, is based. It is the primary drug on which the basic programming decisions for drug infusion system 12 are based.

While one drug may be the primary drug contained in the fluid medication, the medical professional must not overlook the effects of other drugs contained in the fluid medication. If the prescription for the amount of the primary drug in increased, typically by increasing the delivery rate of pump 36, the amount of all other drugs, which are contained in fluid medication, delivered to patient 10 will also be increased. Thus, the medical professional must take into account all of the drugs contained in fluid medication. If the dose for the primary drug is changed, then the dose for all of the drugs will be changed. If the medical professional does not keep track of the affect of modifying the delivery rate on all of the drugs contained in the fluid medication, the patient 10 may receive more or less of the other drugs contained in the fluid medication.

Programmer 20 portion of controller 32 provides an interface between the medical professional and the potentially implanted drug infusion device 14. In particular, programmer 20 provides a medium for data entry into memory 26 of drug infusion device 14 and provides a display for communication of information contained in memory 26 to the medical professional. As noted above, programmer 20 also, preferably, provides computational power to perform the calculations associated with drug infusion system 12.

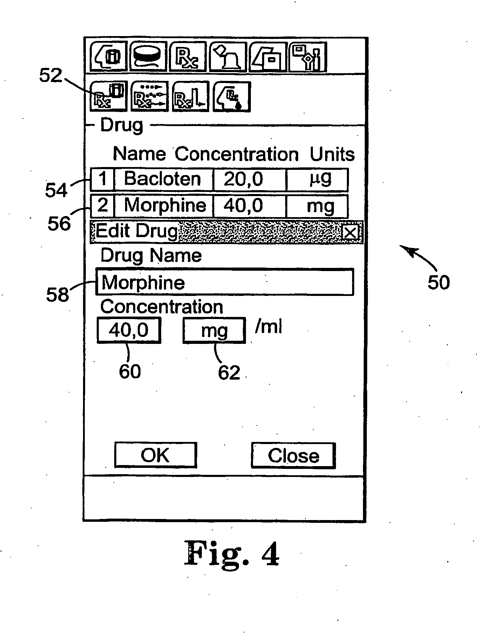

Figure 4 illustrates an "input-output" display 50 on programmer 20 with the drug tab 52 selected. Display 50, associated with drug tab 52, provides a mechanism for the medical professional to input information about the drugs contained in the fluid medication to controller 32. Display 50 also provides a mechanism for the medical professional to learn with what drugs controller 32 has been programmed.

Drug entry display has an entry (54, 56) for each of the multiple drugs contained fluid medication. Entry number 1 (54) contains information on the primary drug including the name of the drug and the concentration of the drug. In the embodiment illustrated in Figure 4, entry 54 contains Baclofen. Entry 54 also contains information about the concentration of the primary drug Baclofen, here listed as 20.0 micrograms. This concentration means the fluid medication has 20.0 micrograms of Baclofen per milliliter of fluid medication. Entry 56 illustrates the entry of information about drug 2, a secondary drug in fluid medication. In the embodiment illustrated in Figure 4, entry 56 contains morphine. The information about secondary drug morphine is expanded from its read-only status at entry 56 to data entry dialog boxes 58, 60 & 62. Dialog box 58 appears to facilitate entry of the actual name of morphine as a secondary drug. This is the spot that the medical professional enters this information. Similarly, dialog box 60 facilitates entry of amount of the concentration of morphine and dialog box 62 facilitates entry of the units associated with the amount of the concentration of morphine. In the example illustrated in Figure 4, secondary drug morphine has a concentration of 40.0 milligrams per milliliter of fluid medication. Once the medical professional is assured that the information contained in dialog boxes 58, 60 & 62 are correct, the "OK" box can be clicked the entry 56 will be updated with the proper information from dialog boxes 58, 60 & 62.

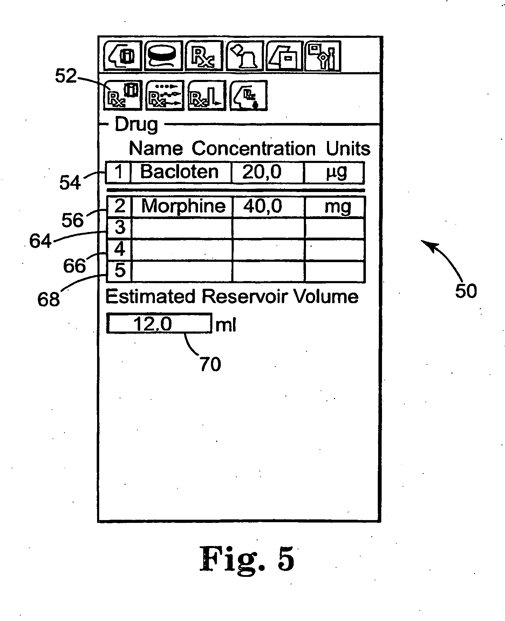

Figure 5 illustrates the appearance of drug entry display 50 with dialog box 58, 60 & 62 closed. Entries 54 and 56 appear as they did in Figure 4. In addition, drug entry display 50 illustrated in Figure 5 contains spaces for entries 64, 66 & 68. Since, only two drugs are contained in fluid medication in the example illustrated in Figure 5, entries 64, 66 & 68 are empty. If, however, fluid medication contained more than two drugs, information about the additional drugs would be contained in entries 64 (if there were a total of three drugs), 64 and 66 (if there were a total of four drugs) and entries 64, 66 and 68 (if there were total of five drugs). Of course, additional drugs could be accommodated with additional entries.

In addition to information about each individual drug contained in fluid medication, display 50 also contains information (entry 70) about the estimated volume of fluid medication contained in reservoir 34. The initial value entered into entry 70 would be the total amount of fluid medication which is, or is to be, inserted into reservoir 34 of drug infusion device 14. After initial entry of the value contained in entry 70, drug infusion device 14 calculates the amount fluid medication which has been delivered by drug infusion device 14 and subtracts that amount from the initial value entered into entry 70. Drug infusion device; via controller 34, then causes the updated amount of fluid medication remaining in reservoir 34 (as calculated above) to be displayed in entry 70.

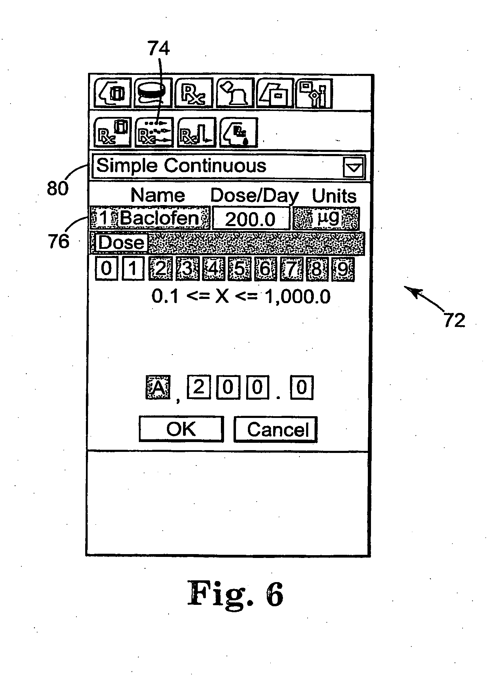

Figure 6 illustrates drug delivery display 72 as selected by drug delivery tab 74. Drug delivery display 72 has been selected, via delivery mode drop-down box 80, to be in "simple continuous" mode, meaning that pump 36 is programmed to delivery fluid medication at a constant rate. Drug delivery display 72 has entry 76 which is indicative of the prescribed dose of the primary drug (entered in drug entry display screen 50). In this part of the example, the primary drug is identified in entry 76 as Baclofen. The data entry in the lower portion of drug delivery display 72 is open and illustrates the entering of a daily dose of Baclofen (drug 1, the primary drug) of 200 micrograms.

The daily dose of the primary drug entered in drug delivery display 72 converted by controller 32, preferably programmer 20, into a drug delivery rate, i.e., the rate at which pump 36 delivers the fluid medication to patient 10, and is transferred to memory unit 26. Controller 32 calculates the rate at which pump 36 delivers fluid medication to patient 10 by converting, if necessary, the daily dose the primary drug into a dose of the primary drug in a smaller unit of time. This amount of dose is then divided by the concentration of the primary drug in the fluid medication contained in reservoir 34 resulting in an amount of the fluid medication to be delivered to patient 10 over that unit of time. Pump 36 is then set to deliver the fluid medication at that rate.

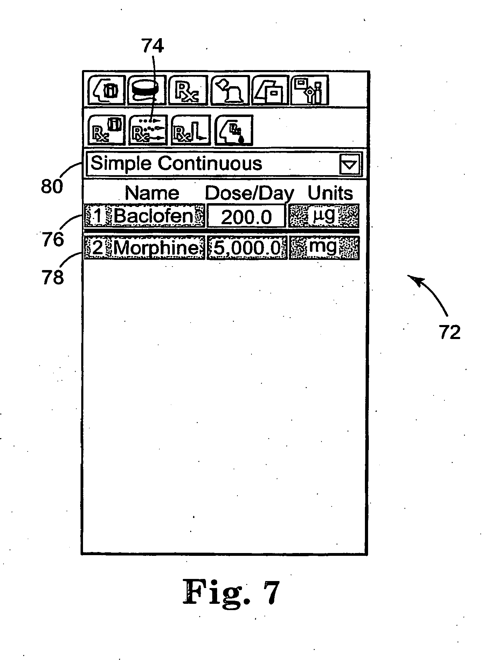

Figure 7 illustrates drug delivery display 72 also as selected by drug delivery tab 74 and also in simple continuous mode as selected by delivery mode drop-down box 80. Drug delivery display 72 illustrated in Figure 7 differs from drug delivery display 72 illustrated in Figure 6 in that the data entry in the lower portion of drug delivery display 72 is now closed, the data for the drug delivery rate for primary drug Baclofen having been entered in entry 76. Closing of the data entry portion allows drug delivery display 72 not only entry 76 for primary drug Baclofen but also allows display entry 78 for secondary drug identified as morphine in this portion of the example.

Drug dose entry 76 displays the secondary drug name, morphine, and the daily dose for the secondary drug, here 5,000 milligrams. In contrast to drug dose entry 76 for primary drug Baclofen, drug dose delivery entry 78 for secondary drug morphine is not directly entered by the medical professional. Since the medical professional has already set the rate at which pump 36 delivers fluid medication to patient 10 via drug dose delivery rate 76, all other drugs in fluid medication will be delivered at that same rate. Hence, drug dose entry 78 is instead an informational display of a calculated daily dose for secondary drug morphine for the medical professional.

The daily dose of secondary drug, morphine, displayed here in entry 78 is calculated by controller 32. The rate at which pump 36 is set to deliver the fluid medication to patient 10 is known by the calculation resulting from the dosing programmed for the primary drug, Baclofen. The rate at which pump 36 is set to deliver fluid medication to patient 10 per unit time is multiplied by the concentration of the secondary drug in the fluid medication contained in reservoir 34. This results in the dose of the secondary drug set to be delivered to patient 10 per unit of time. The dose is then converted into a daily dose simply by adjusting the time scale, if necessary. The resulting daily dose is then displayed in entry 78 of drug dose display 72.

While Figures 6 and 7 have been illustrated with two drugs, a primary drug and a secondary drug, it is to be recognized and understood that more secondary drugs could also be mixed in fluid medication and, hence, also be added as additional entries in drug delivery display 72. A drug contained in fluid medication will have an entry in both drug entry display 50 and drug delivery display 72.

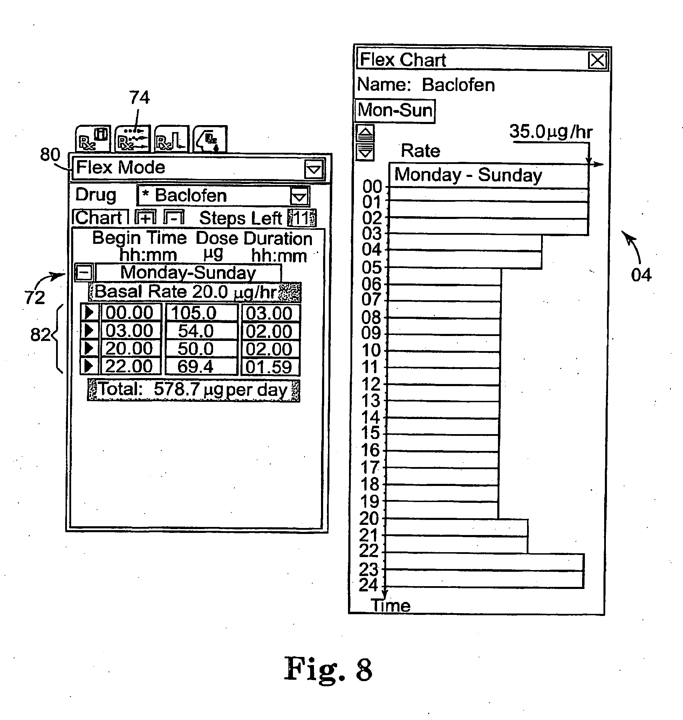

Figure 8 illustrates drug delivery display 72, as selected by drug delivery tab 74, this time illustrating the programming of the delivery of primary drug Baclofen in "flex mode" as selected by drop-down box 80. In flexible mode, the delivery rate for the primary drug can be varied based on multiple time periods, such as particular hours, in a longer time period, such as a day. The variation in drug delivery rate can be entered directly by particular interval step as shown at 82 or can be displayed and/or entered as illustrated in the alternative graphical representation 84.

Again, once the delivery mode and rate for the primary drug has been entered, controller 32 of drug infusion devices. 14 calculates and displays, via drug delivery display 72, the daily dose and drug delivery particulars, if in flexible mode, of a secondary drug or drugs.

By displaying the daily dosage or particular delivery rates for a secondary drug or drugs directly in the display of programmer 20, the medical professional is kept apprised of the affect of dosing decisions based upon the primary drug on dosing for a secondary drug or drugs. The makes programming drug infusion device easier, more straightforward and helps eliminate errors and dosing miscalculations.

## Claims

1. A drug infusion system capable of delivering a fluid medication to a patient at a flow rate under direction of a medical professional, said fluid medication consisting of a plurality of drugs to be administered from a reservoir, each of said plurality of drugs having a concentration, said drug infusion system comprising:
a drug delivery module (14) capable of delivering said fluid medication to said patient from said reservoir; and
a controller (32) allowing said medical professional to specify a dose per unit time of one of said plurality of drugs; **characterised by**
means (20, 26) for determining a flow rate of another of said plurality of drugs as a function of said dose per unit time of said one of said plurality of drugs and said concentration of said another of said plurality of drugs;
said controller communicating said flow rate of said another of said plurality of drugs to said medical professional.

2. A drug infusion system as in claim 1 wherein said drug delivery module is implantable.

3. A drug infusion system as in claim 1 wherein said concentration of each of said plurality of drugs is stored in said drug delivery module.

4. A drug infusion system as in claim 1 wherein said one of said plurality of drugs is a primary drug and wherein said another of said plurality of drugs is a secondary drug.

5. A drug infusion system as in claim 4 which further comprises means for determining a dose of said secondary drug per unit time for said patient as a function of said flow rate and said concentration of said secondary drug.

6. A drug infusion system as in claim 5 wherein said controller communicates said dose for said secondary drug per unit time to said medical professional via a display.

7. A drug infusion system as in claim 4 wherein said concentration of each of said plurality of drugs is stored in said drug delivery module.

## Patentansprüche

1. Medikamenteninfusionssystem, das zur Abgabe einer Fluidmedikation an einem Patienten mit einer Flussrate unter Anleitung eines Mediziners geeignet ist, wobei die Fluidmedikation aus einer Anzahl aus einem Reservoir zu verabreichender Medikamente besteht, wobei jedes aus der Anzahl von Medikamenten eine Konzentration aufweist, wobei das Medikamenteninfusionssystem aufweist:
ein Medikamentenabgabemodul (14), das zur Abgabe der Fluidmedikation an den Patienten aus dem Reservoir geeignet ist; und
ein Steuergerät (32), das es dem Mediziner ermöglicht, eine Dosis pro Zeiteinheit für eines aus der Anzahl von Medikamenten zu spezifizieren; **gekennzeichnet durch**
Mittel (20, 26) zum Bestimmen einer Flussrate eines anderen aus der Anzahl von Medikamenten als einer Funktion der Dosis pro Zeiteinheit des einen aus der Anzahl von Medikamenten und der Konzentration des anderen aus der Anzahl von Medikamenten;
und **dadurch**, dass das Steuergerät die Flussrate des anderen aus der Anzahl von Medikamenten dem Mediziner mitteilt.

2. Medikamenteninfusionssystem nach Anspruch 1, bei dem das Medikamentenabgabemodul implantierbar ist.

3. Medikamenteninfusionssystem nach Anspruch 1, bei dem die Konzentration eines jeden aus der Anzahl von Medikamenten in dem Medikamentenabgabemodul gespeichert ist bzw. wird.

4. Medikamenteninfusionssystem nach Anspruch 1, bei dem das eine aus der Anzahl von Medikamenten ein Primärmedikament ist und bei dem das andere aus der Anzahl von Medikamenten ein Sekundärmedikament ist.

5. Medikamenteninfusionssystem nach Anspruch 4, das ferner Mittel zum Bestimmen einer Dosis des Sekundärmedikaments pro Zeiteinheit für den Patienten als einer Funktion der Flussrate und der Konzentration des Sekundärmedikaments aufweist.

6. Medikamenteninfusionssystem nach Anspruch 5, bei dem das Steuergerät die Dosis pro Zeiteinheit für das Sekundärmedikament dem Mediziner über ein Display mitteilt.

7. Medikamenteninfusionssystem nach Anspruch 4, bei dem die Konzentration eines jeden aus der Anzahl von Medikamenten in den Medikamentenabgabemodul gespeichert ist bzw. wird.

## Revendications

1. Système de perfusion de médicaments capable d'administrer une médication fluide à un patient à un certain taux de flux on taus de flux ou débit sous la direction d'un professionnel médical, ladite médication fluide étant constituée d'une pluralité de médicaments à administrer depuis un réservoir, chaque médicament parmi ladite pluralité de médicaments ayant une concentration, ledit système de perfusion de médicaments comportant :
un module d'administration de médicaments (14) capable d'administrer ladite médication fluide audit patient depuis ledit réservoir ; et
un dispositif de commande (32) permettant au professionnel médical de spécifier une dose par unité de temps d'un médicament parmi ladite pluralité de médicaments ; **caractérisé par**
des moyens (20, 26) pour déterminer un débit d'un autre médicament parmi ladite pluralité de médicaments en fonction de ladite dose par unité de temps dudit médicament parmi ladite pluralité de médicaments et de ladite concentration dudit autre médicament parmi ladite pluralité de médicaments ;
ledit dispositif de commande communiquant ledit débit audit autre médicament parmi ladite pluralité de médicaments audit professionnel médical.

2. Système de perfusion de médicaments selon la revendication 1, dans lequel ledit module d'administration de médicaments est implantable.

3. Système de perfusion de médicaments selon la revendication 1, dans lequel ladite concentration de chaque médicament parmi ladite pluralité de médicaments est stockée dans ledit module d'administration de médicaments.

4. Système de perfusion de médicaments selon la revendication 1, dans lequel ledit un médicament parmi ladite pluralité de médicaments est un médicament principal et dans lequel ledit autre médicament parmi ladite pluralité de médicaments est un médicament secondaire.

5. Système de perfusion de médicaments selon la revendication 4, lequel comporte en outre des moyens pour déterminer une dose dudit médicament secondaire par unité de temps pour ledit patient en fonction dudit débit et de ladite concentration dudit médicament secondaire.

6. Système de perfusion de médicaments selon la revendication 5, dans lequel ledit dispositif de commande communique ladite dose pour ledit médicament secondaire par unité de temps audit profession médical via un écran.

7. Système de perfusion de médicaments selon la revendication 4, dans lequel ladite concentration de chaque médicament parmi ladite pluralité de médicaments est stockée dans ledit module d'administration de médicaments.
